(19) Europäisches Patentamt
European Patent Office
Office européen des brevets

(11) EP 0 726 251 B1

(12) # EUROPÄISCHE PATENTSCHRIFT

(45) Veröffentlichungstag und Bekanntmachung des Hinweises auf die Patenterteilung:
**31.03.1999 Patentblatt 1999/13**

(51) Int Cl.[6]: **C07C 323/58**, A61K 7/09

(21) Anmeldenummer: **96100263.1**

(22) Anmeldetag: **10.01.1996**

(54) **Mittel zur dauerhaften Haarverformung sowie neue haarkeratinreduzierende Cysteinester**

Agents for permanent hair waving as well as hair keratin reducing cycsteine esters

Agents pour la mise en plis permanente des cheveux ainsi que des esters de cystéine réducteurs de la kératine des cheveux

(84) Benannte Vertragsstaaten:
**DE ES FR GB IT**

(30) Priorität: **09.02.1995 DE 19504215**

(43) Veröffentlichungstag der Anmeldung:
**14.08.1996 Patentblatt 1996/33**

(73) Patentinhaber: **Wella Aktiengesellschaft**
**64274 Darmstadt (DE)**

(72) Erfinder:
- **Braun, Hans J., Dr.**
  **CH-3182 Ueberstorf (CH)**
- **Lang, Günther, Dr.**
  **D-64354 Reinheim (DE)**
- **Maresch, Gerhard**
  **D-64295 Darmstadt (DE)**

(56) Entgegenhaltungen:

| | |
|---|---|
| **EP-A- 0 624 572** | **DE-A- 4 336 838** |
| **FR-A- 2 361 864** | **US-A- 5 350 572** |

- **J. PHARM. SCI., Bd. 71, Nr. 5, 1982, Seiten 514-520, XP000569378 N. BODOR ET AL: & DATABASE BEILSTEIN BEILSTEIN INSTITUTE FOR ORGANIC CHEMISTRY, FRANKFURT/MAIN, DE (STN),**
- **POLYM. J. (TOKYO), Bd. 15, Nr. 7, 1983, Seiten 519-524, XP000565707 N. UEYAMA ET AL:**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Mittel zur dauerhaften Haarverformung sowie neue haarkeratinreduzierende Cysteinester.

Für eine schonende dauerhafte Verformung von geschädigtem, insbesondere gebleichtem oder gefärbtem Haar werden bevorzugt schwach sauer bis neutral eingestellte Verformungsmittel verwendet. Hierbei haben sich im Verlauf der letzten 30 Jahre die Thioglykolsäureester als für diesen Zweck am besten geeignete Reduktionsmittel erwiesen.

Eine weitere Möglichkeit zur Verformung von vorgeschädigtem Haar bieten Haarverformungsmittel auf der Basis von Cystein oder Cysteinethylester beziehungsweise deren Salzen, wobei der pH-Wert dieser Mittel vorzugsweise im mildalkalischen Bereich (pH = 7,1 bis 9) liegt.

Dem Vorteil einer haarschonenderen Verformungsbehandlung durch schwach saure bis neutrale Verformungsmittel stehen jedoch eine Reihe von Nachteilen gegenüber.

So besitzen diese Mittel eine im Vergleich zu mildalkalischen Verformungsmitteln auf Thioglykolatbasis geringere Wellwirksamkeit. Aus diesem Grunde ist für die Erzielung einer ausreichenden Umformung die Zufuhr von Wärme, eine Verlängerung der Einwirkungszeiten auf 20 bis 60 Minuten sowie die Verwendung von vergleichsweise dünnen Wicklern erforderlich. Eine Verwendung dieser Verformungsmittel für normales, nicht geschädigtes Naturhaar erscheint aufgrund der auch bei Wärmezufuhr erforderlichen langen Einwirkungszeiten von über 30 Minuten nicht sinnvoll, so daß die Anwendung von schwach sauer bis neutral eingestellten Verformungsmitteln bisher in der Regel auf vorgeschädigtes, leicht verformbares Haar beschränkt blieb.

Ein weiterer erheblicher Nachteil von sauren Haarverformungsmitteln ist die schlechte Augen- und Hautverträglichkeit sowie die sensibilisierende Wirkung der Thioglykolsäureester.

Trotz einer Vielzahl von Versuchen konnte bisher die sensibilisierende Wirkung von sauren Haarverformungsmitteln nicht entscheidend verringert werden. Als Alternative wurden daher mildalkalische (pH = 7,1 bis 9) Haarverformungsmittel vorgeschlagen, die als keratinreduzierenden Wirkstoff Cystein oder dessen Salze enthalten.

Diese Haarverformungsmittel weisen jedoch ebenfalls eine Reihe von Nachteilen auf. So besitzt Cystein nur eine schwache Verformungswirksamkeit sowie eine geringe Stabilität. Wenn cysteinhaltige Verformungsmittel auf das Haar aufgebracht werden, wird das Cystein durch den Luftsauerstoff schnell zu in Wasser schwer löslichem Cystin oxidiert, welches sich in Form eines störenden, schwer entfernbaren weißen Belages auf dem Haar ablagert.

Die Oxidationsanfälligkeit des Cysteins beziehungsweise seiner Salze macht den Einsatz von Cystein-Dauerwellen nahezu unmöglich und kann derzeit, wenn überhaupt, nur durch den Zusatz von geeigneten Co-Reduktionsmitteln verbessert werden.

Der Erfindung liegt daher die Aufgabe zugrunde, ein Mittel zur dauerhaften Haarverformung zur Verfügung zu stellen, das sowohl im sauren als auch schwach alkalischen Bereich (pH-Wert = 4,5 bis 9,0) eine schonende und gleichmäßige Umformung ermöglicht, kein oder aber nur ein geringes Allergiepotential aufweist und bei dem darüber hinaus keine störenden Ablagerungen oder Beläge auf dem Haar entstehen.

Überraschenderweise wurde nunmehr gefunden, daß mit Mitteln zur dauerhaften Verformung von Haaren auf der Basis von bestimmten Cysteinestern eine gleichmäßige Umformung sowohl von geschädigtem als auch ungeschädigtem Haar möglich ist, ohne daß hierbei die bei den bisher für die Haarverformung verwendeten haarkeratinreduzierenden Esterverbindungen häufig beobachteten allergischen Hautreaktionen auftreten oder die bei cysteinhaltigen Dauerverformungsmitteln beobachteten störenden Beläge auf dem Haar entstehen.

Gegenstand der vorliegenden Erfindung ist daher ein Mittel zur dauerhaften Verformung von Haaren auf der Basis eines keratinreduzierenden Wirkstoffes, welches als keratinreduzierenden Wirkstoff mindestens einen Cysteinester der allgemeinen Formel (I)

$$HS\text{-}CH_2\text{-}CH(NH_2)\text{-}COO\text{-}CH_2\text{-}CH_2\text{-}R \qquad (I),$$

mit R gleich einer Hydroxygruppe, einer Alkoxygruppe, einer Poly(oxyalkyl)gruppe oder einer Hydroxypoly(oxyalkyl) gruppe,oder dessen Säureadditionssalz enthält.

In der Formel (I) bedeutet die Restgruppe R vorzugsweise eine Hydroxygruppe;
eine ($C_1$-$C_6$-Alkoxy)gruppe; eine Poly(oxyalkyl)gruppe der Formel $[O\text{-}(C_1\text{-}C_6\text{-Alkyl})]_nH$ in der der Polymerisationsgrad n gleich 2 bis 20, besonders bevorzugt gleich 2 bis 10, ist; oder eine Hydroxy-poly(oxyalkyl)gruppe der Formel $[O\text{-}(C_1\text{-}C_6\text{-Alkyl})]_mOH$ in der der Polymerisationsgrad m gleich 1 bis 20, besonders bevorzugt gleich 2 bis 10, ist. Der Ausdruck ($C_1$-$C_6$-Alkyl) steht hierbei für eine Alkylgruppe mit 1 bis 6 Kohlenstoffatomen, während die Bezeichnung ($C_1$-$C_6$-Alkoxy) für eine Alkoxygruppe steht, deren Alkylgruppe 1 bis 6 Kohlenstoffatome enthält, wobei die Alkylgruppe jeweils verzweigt oder unverzweigt sein kann.

Als Säureadditionssalze können alle physiologisch verträglichen Additionssalze mit organischen oder anorganischen Säuren, wie zum Beispiel Hydrochloride, Sulfate, Hydrobromide oder Acetate, verwendet werden.

Als Cysteinester der Formel (I) sind insbesondere der L-Cystein-(2-hydroxyethyl)ester und der L-Cystein-(2-methoxyethyl)ester sowie deren Hydrochloride zu nennen.

Zwar ist es möglich die Cysteinester der Formel (I) gemeinsam mit anderen keratinreduzierenden Wirkstoffen - wie zum Beispiel Thioglykolsäure, Thiomilchsäure, 3-Hydroxy-2-mercaptopropionsäure, Cysteamin und Cysteaminderivaten oder Cystein und Cysteinderivaten - zu verwenden, jedoch ist die Verwendung des Cysteinesters der Formel (I) als alleinigem keratinreduzierenden Wirkstoff (das heißt ohne Zusatz anderer keratinreduzierender Wirkstoffe) besonders bevorzugt.

Der Cysteinester der Formel (I) oder dessen Säureadditionssalz wird in dem gebrauchsfertigen erfindungsgemäßen Mittel zur dauerhaften Haarverformung in einer Menge von 4 bis 30 Gewichtsprozent, vorzugsweise in einer Menge von 8 bis 28 Gewichtsprozent, eingesetzt.

Das gebrauchsfertige erfindungsgemäße Verformungsmittel besitzt einen pH-Wert von 4,5 bis 9,0, vorzugsweise von 6,0 bis 8,5.

Das Verformungsmittel kann sowohl in Form einer wäßrigen Lösung oder einer Emulsion als auch in verdickter Form auf wäßriger Basis, insbesondere als Gel, Creme oder Paste vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; oder Alkohole, wie beispielsweise Ethanol, Propanol, Isopropanol oder Glycerin; Lösungsvermittler; Stabilisatoren; Puffersubstanzen; Parfümöle; Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 2 bis 30 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Da Carbonsäureester in wäßrigem Medium nur für begrenzte Zeit haltbar sind und, insbesondere im alkalischen Bereich, leicht hydrolysieren können, wird der Cysteinester der Formel (I) vorzugsweise in wasserfreier Form abgepackt und das Verformungsmittel erst unmittelbar vor Gebrauch durch Vermischen der den Cysteinester enthaltenden Komponente mit einer oder mehreren weiteren Komponenten hergestellt.

Je nach Konfektionierung kann das erfindungsgemäße Mittel hierbei in Form eines Zwei- oder Dreikomponenten-Präparates vorliegen.

So ist das erfindungsgemäße Mittel zum Beispiel durch Vermischen zweier Komponenten erhältlich, von denen die erste Komponente das oder die Alkalisierungsmittel, beispielsweise ein Alkalicarbonat, Ammoniumcarbonat, Alkalihydrogencarbonat oder Ammoniumhydrogencarbonat, sowie die vorstehend genannten kosmetischen Zusatzstoffe und Wasser enthält, während die zweite, wasserfreie Komponente den Cysteinester der Formel (I) enthält.
Ebenfalls ist es möglich, das erfindungsgemäße Mittel in Form eines Dreikomponenten-Präparates abzupacken, wobei eine Komponente einen Teil der vorstehend genannten kosmetischen Zusatzstoffe sowie Wasser, eine zweite, wasserfreie Komponente den Cysteinester der Formel (I) und die dritte Komponente Parfümöle, Lösungsvermittler und Pflegestoffe in wäßriger Lösung oder wasserfreier Form enthält.

Bei allen Ausführungsformen des erfindungsgemäßen Mittels können die vorstehend genannten kosmetischen Zusatzstoffe sowohl in der wäßrigen als auch in der/den nichtwäßrigen Komponente(n) enthalten sein.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte, gleichmäßige Umformung vom Haaransatz bis zur Haarspitze.

Die dauerhafte Verformung der Haare wird in der üblichen Weise durchgeführt, indem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet.

In einer bevorzugten Ausführungsform wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt („fixiert"). Das Nachbehandlungsmittel wird, je nach Haarfülle, vorzugsweise in einer Menge von 80 bis 100 Gramm verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Wasserstoffperoxid.

Die Konzentration des Oxidationsmittels ist in Abhängigkeit von der Anwendungszeit (in der Regel 2 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die in dem erfindungsgemäßen Mittel zur Verformung der Haare enthaltenden Cysteinester der Formel (I) können in üblicher Weise durch Veresterung von Cystein in Gegenwart eines sauren Katalysators, beispielsweise Schwefelsäure, Salzsäure oder Benzolsulfonsäure, mit einem geeigneten Alkohol, vorzugsweise unter Wärmeeinwirkung, erhalten werden.

Die vorgenannten Cysteinester der Formel (I) besitzen eine hohe Wellwirksamkeit bei sauren bis leicht alkalischen pH-Werten, sind nahezu geruchsneutral und leicht wasserlöslich und weisen eine hervorragende physiologische Verträglichkeit sowie eine gute Stabilität in Wasser auf.

Cysteinester der Formel (I) mit R = Poly(oxyalkyl)gruppe sind von Ueyama et al. im Polymer Journal, Vol. 15, No. 7, Seiten 519 bis 524 (1983) beschrieben.

Ein weiterer Gegenstand der vorliegenden Anmeldung sind neue Ester des Cysteins („Cysteinester") gemäß Anspruch 8, von denen der L-Cystein-(2-hydroxyethyl)ester (II) und der L-Cystein-(2-methoxyethyl)ester (III) oder deren Säureadditionssalze, alleine oder in Kombination, besonders bevorzugt sind.

$$HS\text{-}CH_2\text{-}CH(NH_2)\text{-}COO\text{-}CH_2CH_2OH \qquad (II)$$

$$HS\text{-}CH_2\text{-}CH(NH_2)\text{-}COO\text{-}CH_2CH_2OCH_3 \qquad (III)$$

Die Cysteinester der Formel (I) können in Analogie zu den nachfolgenden Herstellungsbeispielen durch eine säurekatalysierte Veresterung von Cystein mit den entsprechenden Hydroxyverbindungen (Ethylenglykol beziehungsweise Ethylenglykolderivaten) hergestellt werden.

Die nachfolgenden Beispiele sollen den Gegenstand der Erfindung näher erläutern, ohne ihn hierauf zu beschränken.

**Beispiele für Haarverformungsmittel**

**Beispiel 1: Zweikomponenten-Haarverformungsmittel**

| | | |
|---|---|---|
| Komponente 1 | 3,0 g | Harnstoff |
| | 1,0 g | Octylphenol, mit 20 Mol Ethylenoxid oxethyliert |
| | 0,8 g | Ammoniak (25-prozentige wäßrige Lösung) |
| | 0,5 g | Parfümöl |
| | 0,3 g | Ammoniumhydrogencarbonat |
| | 94,4 g | Wasser, vollentsalzt |

(fortgesetzt)

| | | |
|---|---|---|
| | 100,0 g | |
| Komponente 2 | 70,0 g | L-Cystein-(2-hydroxyethyl)ester |
| | 30,0 g | Glycerin |
| | 100,0 g | |

Vor dem Gebrauch werden 70 Gramm der Komponente 1 und 30 Gramm der Komponente 2 zu einem Haarverformungsmittel mit einem pH-Wert von 6,3 vermischt.

Leicht vorgeschädigtes Haar wird mit einem Shampoo gewaschen und mit Wasser gründlich gespült. Anschließend wird das frottierte Haar auf Wickler mit einem Durchmesser von 8 Millimetern gewickelt, mit dem Haarverformungsmittel gleichmäßig befeuchtet und mit einer Plastikhaube abgedeckt. Nach einer Einwirkzeit von 15 Minuten bei 40°C wird die Plastikhaube entfernt, das Haar mit Wasser gespült und mit 100 Gramm einer 3-prozentigen wäßrigen Wasserstoffperoxidlösung oxidativ nachbehandelt.

Nach Entfernung der Wickler werden die Haare erneut mit Wasser gespült, zur Frisur gelegt und sodann getrocknet.

Als Ergebnis dieser Behandlung wird eine gleichmäßige, natürlich wirkende Umformung der Haare vom Haaransatz bis zu den Haarspitzen erhalten.

**Beispiel 2: Zweikomponenten-Dauerverformungsmittel**

| | | |
|---|---|---|
| Komponente 1 | 2,0 g | Dipropylenglykolmonomethylether |
| | 1,0 g | Ammoniak (25-prozentige wäßrige Lösung) |
| | 0,3 g | Kokosfettalkohol, mit 10 Mol Ethylenoxid oxethyliert |
| | 0,3 g | Parfümöl |
| | 0,2 g | Diammoniumdithiodiglykolat |
| | 96,2 g | Wasser, vollentsalzt |
| | 100,0 g | |
| Komponente 2 | 60,0 g | L-Cystein-(2-hydroxyethyl)ester |
| | 30,0 g | Glycerin |
| | 10,0 g | N-Acetylcysteamin |
| | 100,0 g | |

80 Gramm der Komponente 1 werden mit 40 Gramm der Komponente 2 zu einem gebrauchsfertigen Haarverformungsmittel vom pH 6,5 vermischt.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 20 Minuten bei Raumtemperatur wird das Haar mit Wasser gründlich gespült und sodann mit 80 Gramm einer 3-prozentigen wäßrigen Wasserstoffperoxid-Lösung oxidativ nachbehandelt. Nach Entfernung der Wickler werden die Haare erneut mit Wasser gespült, zur Wasserwelle gelegt und anschließend getrocknet.

Das so umgeformte Haar besitzt eine über die gesamte Haarlänge gleichmäßige Krause, die mit der durch Behandlung mit mildalkalischen Haarverformungsmittel erzielten Krause vergleichbar ist.

**Beispiel 3: Dreikomponenten-Dauerverformungsmittel**

| | | |
|---|---|---|
| Komponente 1 | 1,9 g | 1,2-Propylenglykol |
| | 1,0 g | Glycerinindiacetat |
| | 0,8 g | Kokosfettalkohol, mit 10 Mol Ethylenoxid oxethyliert |
| | 0,3 g | Parfümöl |

(fortgesetzt)

| | | |
|---|---|---|
| Komponente 2 | 4,0 g | Harnstoff |
| | 1,5 g | Ammoniak (25-prozentige wäßrige Lösung) |
| | 1,2 g | Ammoniumhydrogencarbonat |
| | 89,3 g | Wasser |
| Komponente 1+2 | 100,0 g | |
| Komponente 3 | 80,0 g | L-Cystein-(2-methoxyethyl)ester |
| | 20,0 g | Glycerin |
| | 100,0 g | |

Komponente 1 wird unmittelbar vor der Anwendung in Komponente 2 gelöst. Anschließend werden 70 Gramm dieser Lösung mit 30 Gramm der Komponente 3 vermischt. Der pH-Wert des gebrauchsfertigen Dauerverformungsmittels beträgt 6,8.

Ein Haar von ungleichmäßiger Haarqualität wird gewaschen, mit einem Handtuch frottiert und auf Wickler mit einem Durchmesser von 6 Millimetern gewickelt. Anschließend wird das Haar mit dem vorstehend beschriebenen gebrauchsfertigen Dauerverformungsmittel gleichmäßig durchfeuchtet.

Nach der Einwirkungszeit von 15 Minuten bei 40°C wird das Haar mit Wasser gespült und sodann mit 80 g einer 3-prozentigen wäßrigen Wasserstoffperoxidlösung oxidativ nachbehandelt. Nach dem Entfernen der Wickler werden die Haare erneut mit Wasser gespült, zur Wasserwelle gelegt und anschließend getrocknet.

Als Ergebnis dieser Behandlung wird ein gleichmäßig verformtes Haar mit hoher Sprungkraft und Elastizität erhalten.

**Herstellungsbeispiele:**

**Beispiel 4: L-Cystein-(2-hydroxyethyl)ester•HCl**

$$HS\text{-}CH_2\text{-}CH(NH_2)\text{-}COOH \xrightarrow[HCl\,(3n)]{HO\text{-}CH_2CH_2\text{-}OH} HS\text{-}CH_2\text{-}CH(NH_2)\text{-}COO\text{-}CH_2CH_2OH\bullet HCl$$

12,1 g (0,1 mol) L-Cystein werden in 100 ml Ethylenglykol mit 50 ml 3n Salzsäure 18 Stunden lang unter Rückfluß gekocht. Anschließend werden unter Normaldruck 50 ml Wasser und sodann bei vermindertem Druck das Ethylenglykol beziehungsweise dessen Azeotrop mit Wasser abdestilliert. Der zurückbleibende Rückstand wird in 150 ml Tetrahydrofuran (THF) aufgenommen. Das erhaltene Gemisch wird sodann zum Sieden erhitzt. Nach dem Abkühlen wird der Feststoff abgesaugt, mit Tetrahydrofuran gewaschen und im Exsikkator getrocknet.

Es werden 17,8 (88% der theoretischen Ausbeute) eines beigefarbenen, kristallinen Produktes erhalten, welches bei 152-157°C schmilzt.

CHN-Analyse: $C_5H_{11}NO_3S\bullet HCl$

| (MG = 201,67) | % C | % H | % N |
|---|---|---|---|
| berechnet | 29,78 | 6,00 | 6,95 |
| gefunden | 29,81 | 5,99 | 6,91 |

$^1$H-NMR($D_2O$): δ = 4,33 (t;1H); 4,22 (m;2H); 3,68 (m;2H); 3,08 (m;1H)

FAB-MS (Matrix = Glycerin): m/z = 166(M+1); 134; 122; 93; 76

**Beispiel 5: L-Cystein-(2-methoxyethyl)ester•HCl**

$$HS\text{-}CH_2\text{-}CH(NH_2)\text{-}COOH \xrightarrow[HCl\,(3n)]{HO\text{-}CH_2CH_2\text{-}OCH_3} HS\text{-}CH_2\text{-}CH(NH_2)\text{-}COO\text{-}CH_2CH_2OCH_3\bullet HCl$$

12,1 g (0,1 mol) L-Cystein werden gemeinsam mit 100 ml Methoxyethanol und 50 ml 3-normaler Salzsäure 18 Stunden lang unter Rückfluß erwärmt.

Anschließend werden das bei der Reaktion entstandene Wasser und das Methoxyethanol bei vermindertem Druck abdestilliert.

Der hierbei erhaltene weiße Rückstand wird in 100 ml Ethanol aufgenommen und sodann mit Diethylether in mehreren Fraktionen wieder ausgefällt.

Die erste Fraktion enthält 8,0 g L-Cystein•HCl, während die zweite Fraktion 1,0 g L-Cystein-(2-methoxyethyl)-ester•HCl enthält, welches mit geringen Mengen an L-Cystein•HCl verunreinigt ist.

Ausbeute: 1,0 g (4,6% der theoretischen Ausbeute) L-Cystein-(2-methoxyethyl)ester

FAB-MS (Matrix = Glycerin): m/z = 180(M+1); 166; 122; 93;

Alle in der vorliegenden Anmeldung enthaltenen Prozentangaben stellen, soweit nicht anders angegeben, Gewichtsprozent dar.

## Patentansprüche

1. Mittel zur dauerhaften Verformung von Haaren auf der Basis eines keratinreduzierenden Wirkstoffes, dadurch gekennzeichnet, daß es als keratinreduzierenden Wirkstoff mindestens einen Cysteinester der allgemeinen Formel (I)

$$HS\text{-}CH_2\text{-}CH(NH_2)\text{-}COO\text{-}CH_2\text{-}CH_2\text{-}R \qquad (I),$$

mit R gleicheiner Hydroxygruppe, einer Alkoxygruppe oder einer Poly(oxyalkyl)gruppe oder einer Hydroxypoly (oxyalkyl)gruppe oder dessen Säureadditionssalz enthält.

2. Mittel nach Anspruch 1, dadurch gekennzeichnet, daß es einen Cysteinester der allgemeinen Formel (I), mit R gleich einer Hydroxygruppe;

   einer ($C_1$-$C_6$-Alkoxy)gruppe; einer Poly(oxyalkyl)gruppe der Formel $[O\text{-}(C_1\text{-}C_6\text{-Alkyl})]_nH$, mit n gleich 2 bis 20; oder einer Hydroxy-poly(oxyalkyl)gruppe der Formel $[O\text{-}(C_1\text{-}C_6\text{-Alkyl})]_mOH$ mit m gleich 1 bis 20, oder dessen Säureadditionssalz enthält.

3. Mittel nach Anspruch 1 oder 2, dadurch gekennzeichnet, daß der Cysteinester der Formel (I) der L-Cystein-(2-hydroxyethyl)ester, der L-Cystein-(2-methoxyethyl)ester oder deren Hydrochlorid ist.

4. Mittel nach einem der Ansprüche 1 bis 3, dadurch gekennzeichnet, daß es als alleinigen keratinreduzierenden Wirkstoff einen Cysteinester der Formel (I) enthält.

5. Mittel nach einem der Ansprüche 1 bis 4, dadurch gekennzeichnet, daß der Cysteinester der Formel (I) in dem gebrauchsfertigen Mittel in einer Menge von 4 bis 30 Gewichtsprozent enthalten ist.

6. Mittel nach einem der Ansprüche 1 bis 5, dadurch gekennzeichnet, daß der pH-Wert des gebrauchsfertigen Mittels 4,5 bis 9,0 beträgt.

**7.** Mittel nach einem der Ansprüche 1 bis 6, dadurch gekennzeichnet, daß es vor der Anwendung durch Vermischen von zwei oder drei Komponenten, wobei die den Cysteinester der Formel (I) enthaltende Komponente wasserfrei ist, hergestellt wird.

**8.** Cysteinester der allgemeinen Formel

$$HS\text{-}CH_2\text{-}CH(NH_2)\text{-}COO\text{-}CH_2\text{-}CH_2\text{-}R' \qquad (A),$$

wobei R' eine Hydroxygruppe, eine Alkoxygruppe oder eine Hydroxypoly(oxyalkyl)gruppe bedeutet, und dessen Säureadditionssalze.

**9.** L-Cystein-(2-methoxyethyl)ester.

**10.** L-Cystein-(2-hydroxyethyl)ester.

**11.** L-Cystein-(2-methoxyethyl)ester•HCl.

**12.** L-Cystein-(2-hydroxyethyl)ester•HCl.

**Claims**

**1.** Agent for the permanent shaping of hair based on a keratin-reducing active substance, characterised in that it contains as the keratin-reducing active substance at least one cysteine ester of the general formula (I)

$$HS\text{-}CH_2\text{-}CH(NH_2)\text{-}COO\text{-}CH_2\text{-}CH_2\text{-}R \qquad (I)$$

wherein R is a hydroxy group, an alkoxy group or a poly(oxyalkyl) group or a hydroxypoly(oxyalkyl) group or its acid addition salt.

**2.** Agent according to Claim 1, characterised in that it contains a cysteine ester of the general formula (I) wherein R is a hydroxy group; a ($C_1$-$C_6$ alkoxy) group; a poly(oxyalkyl) group of the formula $[O\text{-}(C_1\text{-}C_6\ alkyl)]_nH$, wherein n is 2 to 20; or a hydroxypoly(oxyalkyl) group of the formula $[O\text{-}(C_1\text{-}C_6\ alkyl)]_mOH$, wherein m is 1 to 20, or its acid addition salt.

**3.** Agent according to Claim 1 or 2, characterised in that the cysteine ester of formula (I) is the L-cysteine-(2-hydroxyethyl) ester, the L-cysteine-(2-methoxyethyl) ester or their hydrochloride.

**4.** Agent according to any one of Claims 1 to 3, characterised in that it contains a cysteine ester of the formula (I) as the sole keratin-reducing active substance.

**5.** Agent according to any one of Claims 1 to 4, characterised in that the cysteine ester of formula (I) is present in the ready-for-use agent in an amount of from 4 to 30 % by weight.

**6.** Agent according to any one of Claims 1 to 5, characterised in that the pH value of the ready-for-use agent is from 4.5 to 9.0.

**7.** Agent according to any one of Claims 1 to 6, characterised in that it is prepared prior to use by mixing two or three components, the component containing the cysteine ester of formula (I) being anhydrous.

**8.** Cysteine esters of the general formala (A)

$$HS\text{-}CH_2\text{-}CH(NH_2)\text{-}COO\text{-}CH_2\text{-}CH_2\text{-}R' \qquad (A),$$

wherein R' is a hydroxy group, an alkoxy group or a hydroxypoly(oxyalkyl) group and their acid addition salts.

**9.** L-cysteine-(2-methoxyethyl) ester.

**10.** L-cysteine-(2-methoxyethyl) ester.

**11.** L-cysteine-(2-methoxyethl) ester•HCL.

**12.** L-cysteine-(2-hydroxyethyl) ester•HCl.

**Revendications**

**1.** Agent de déformation permanente des cheveux, à base d'additif réducteur de la kératine, caractérisé en ce qu'il contient comme additif réducteur de la kératine au moins un ester cystéique de formule générale (I)

$$HS\text{-}CH_2\text{-}CH(NH_2)\text{-}COO\text{-}CH_2\text{-}CH_2\text{-}R \qquad (I),$$

R étant un groupe hydroxy, un groupe alkoxy ou un groupe poly(oxyalkyl) ou un groupe hydroxy-poly(oxyalkyl) ou son sel additif acide.

**2.** Agent selon la revendication 1, caractérisé en ce qu'il contient un ester cystéique de formule générale (I), R étant un groupe hydroxy;

un groupe ($C_1$-$C_6$-alkoxy); un groupe poly(oxyalkyl) de formule $[O\text{-}(C_1\text{-}C_6\text{-alkyl})]_nH$, n étant compris dans la plage allant de 2 à 20; ou un groupe hydroxy-poly(oxyalkyl) de formule $[O\text{-}(C_1\text{-}C_6\text{-alkyl})]_mOH$, m étant compris dans la plage allant de 1 à 20, ou son sel additif acide.

**3.** Agent selon la revendication 1 ou 2, caractérisé en ce que l'ester cystéique de la formule (I) est l'ester L-cystéine-2-(hydroxyéthyl), l'ester L-cystéine-(2-methoxy-éthyl) ou leur hydrochlorure.

**4.** Agent selon l'une des revendications 1 à 3, caractérisé en ce qu'il contient comme seul additif réducteur de la kératine un ester cystéique de la formule (I).

**5.** Agent selon l'une des revendications 1 à 4, caractérisé en ce que l'ester cystéique de la formule (I) est contenu dans l'agent prêt à l'emploi en une quantité comprise dans la plage allant de 4 à 30 pour-cent en poids.

**6.** Agent selon l'une des revendications 1 à 5, caractérisé en ce que la valeur du pH de l'agent prêt à l'emploi se situe dans la plage allant de 4,5 à 9,0.

**7.** Agent selon l'une des revendications 1 à 6, caractérise en ce qu'il préparé avant l'utilisation en mélangeant deux ou trois composants, le composant contenant l'ester cystéique de la formule (I) étant exempt d'eau.

**8.** Esters cystéiques de la formule générale (A)

$$HS\text{-}CH_2\text{-}CH(NH_2)\text{-}COO\text{-}CH_2\text{-}CH_2\text{-}R' \qquad (A),$$

R' étant un groupe hydroxy, un groupe alkoxy ou un groupe hydroxy-poly(oxyalkyl) et leurs sels additifs acides.

**9.** L-cystéine-(2-méthoxyéthyl)ester.

**10.** L-cystéine-(2-hydroxyéthyl)ester.

**11.** L-cystéine-(2-méthoxyéthyl)ester · HCl.

**12.** L-cystéine-(2-hydroxyéthyl)ester · HCl.